# EUROPEAN PATENT APPLICATION

(11) **EP 4 703 709 A1**
(43) Date of publication of application: **04.03.2026**
(21) Application number: 25190050.2
(22) Date of filing: 17.07.2025
(51) Int. Cl.: G01N 21/15, G01N 21/31, G01N 21/53, G01N 21/64, G01N 3/18

(54) **EQUIPMENT FOR MOLECULAR SPECTROPHOTOMETRY AND FLUORIMETRY USING LED TECHNOLOGY**

(30) Priority: 30.08.2024 ES 202430689
(71) Applicant: Universidad Politecnica de Cartagena, 30202 Cartagena (Murcia) (ES)
(72) Inventor: CARRERES PRIETO, Daniel, 30202 Cartagena (ES); GARCÍA BERMEJO, Juan Tomas, 30202 Cartagena (ES)
(74) Representative: Pons IP

(57) **Abstract**

The present invention relates to an equipment comprising a modular structure (1) on which several subsystems are arranged:
- a wavelength generation (12) subsystem,
- a fluorimetric analysis subsystem with an extended range of 90° and 270° (13),
- a scattered light selection between 90° and 270° (90)
- a control electronics (14) subsystem,
- a power supply and plunger lift motors (16)
further comprising an analysis syringe (22) provided with three cleaning means, namely:
∘ mechanical cleaning means
∘ cleaning means consisting of passing clean water through the interior of the analysis syringe (22)
∘ cleaning means for cleaning the inner walls of the analysis walls by means of UV LED lights.

It allows the equipment to be self-cleaning, to perform simultaneous spectrophotometry and fluorimetry, continuous real-time analysis of the pollutant load and prediction of pollutants.

## Description

### OBJECT OF THE INVENTION

The object of the present invention is, as the title of the invention states, an improved apparatus for carrying out a spectrofluorimetric analysis simultaneously and continuously with a single apparatus.

The present invention is characterised by the special design and configuration of each and every one of the elements of the equipment such that it allows the simultaneous measurement and analysis with the same equipment of the spectrophotometric and fluorimetric response to light received from LEDs, in addition to the equipment not being conditioned by the geometry of the sample test tubes used. It also allows continuous, real-time analysis with the ability to self-clean in three different ways, as well as means for adjusting the tightness of the plunger of the analysis syringe.

The equipment also allows the possibility of varying the intensity or brightness level of each incoming LED light or excitation light depending on the water load. In other words, depending on the sample load, the reference brightness level of the LED can be varied, in order to have a better characterisation of the study parameter of the samples.

The equipment improves fluorimetric analysis as it measures the fluorimetric response of scattered light with respect to two different angles, 90° and 270°. This improves the reflected light collection capacity and the fluorophore capacity of the samples in two different positions and not only at 90° as is usual with other existing equipment.

Therefore, the present invention is circumscribed within the field of spectrophotometric and fluorimetric characterisation devices or equipment on water samples, specifically, in the present invention, on waste water samples.

### BACKGROUND OF THE INVENTION

Spectrophotometry is one of the most widely used experimental techniques for the specific detection of molecules. It is characterised by its accuracy, sensitivity and applicability to molecules of different nature (contaminants, biomolecules, etc.) and state of aggregation (solid, liquid, gas).

In the state of the art, utility model ES1273069U discloses a device for characterising the pollutant load present in waste water, comprising a rotating wavelength selector disc which internally comprises a disc with a plurality of diodes, wherein this disc comprises a self-positioning system, it also comprises a vertical displacement mechanism, and a tower comprising a vertical cavity, a circular channel where at one of its ends there is an opening coinciding with a through hole of the circular support that houses a diode.

Fluorimetry is a method of analysis used to measure the intensity of radiation emitted by a material when excited mainly at wavelengths in the ultraviolet region as well as at wavelengths in the near-ultraviolet and visible spectra.

Equipment is available for performing spectrophotometry and fluorimetry, however, for performing fluorimetry they use a CCD array which is a charge-coupled device (also known as a CCD) which is an integrated circuit containing a number of linked or coupled capacitors. Under the control of an internal circuit, each capacitor can transfer its electrical charge to one or more of the capacitors next to it on the printed circuit board - in short, it is a sensor with tiny photoelectric cells that record the image.

The state-of-the-art fluorimetry embodiment requires more complex electronics and higher power consumption.

Furthermore, all spectrophotometry and fluorimetry equipment on the market is only valid for a certain type of specimen geometry and is therefore limited in its functionality. Patent ES2948924 is known in the state of the art describing a spectrophotometry device comprising: A visible and infrared wavelength generator block, a UV wavelength generation block, a variable path length sample containment and analysis block and a fluorimetric analysis block, however, said equipment has aspects that could be improved: the possibility of self-cleaning the equipment in different ways, the disinfection of the interior of the analysis block, as well as the improvement of the reliability of the measurement of the scattered light response for fluorimetry.

Therefore, the object of the present invention is to overcome the aforementioned drawbacks of spectrophotometry with electronic means, particularly the fact that the equipment, specifically the analysis unit, can be self-cleaning, as well as the disinfection of its interior, by developing equipment such as that described below, which is essentially set out in the first claim.

### DESCRIPTION OF THE INVENTION

The object of the present invention is included in its essential nature in the independent claim and the different embodiments are included in the dependent claims.

The present invention relates to a device that analyses both the spectral and fluorimetric response of samples manually fed into the device being able to perform both analyses simultaneously. The equipment works in the wavelength range known as ultraviolet and visible UV-Vis.

The equipment is controlled by a computer via a USB connection.

The sample is introduced by the user through the upper portion of the equipment, which is isolated from the outside light by means of a black hinged lid, in order to avoid alterations in the analysis.

The invention can automatically adapt to samples of different path lengths as well as samples of different heights. The path length is understood as the distance that light passes through the sample to end up emitting an intensity of light, direct or reflected, which is recorded. That is, the path length is the width of the sample specimen to be traversed.

The equipment covered by the invention comprises:
- a wavelength generation subsystem,
- a fluorimetric analysis subsystem,
- a control electronics subsystem,
- a power supply sub-system and plunger lift motors
- an analysis syringe provided with three cleaning media, namely:
   ∘ means of mechanical cleaning consisting of vertical displacement of the analysis syringe in association with vertical positioning means of the mechanical cleaning mechanism of the syringe.
   ∘ cleaning means consisting of passing clean water through the interior of the analysis syringe
   ∘ cleaning means for cleaning the inner walls of the analysis walls by means of UV LED lights in combination with plunger tensioning means

The modular structure comprises a series of platforms interconnected by means of threaded rods, with vertical spacers (3) arranged between the interconnected platforms, defining modular spaces which are covered by rectangular closing platforms.

The mechanical cleaning means for cleaning the analysis syringe (22) comprise a lifting platform vertically movable along threaded spindles driven by lifting motors mounted at the upper end of the threaded spindles by means of couplings.

For the precise arrangement of the lifting platform, neodymium magnets are used on the lifting platform for correct vertical positioning and Hall-effect based limit switch blocks with an upper limit switch and a lower limit switch.

The means of cleaning by means of clean water inside the analysis syringe is based on the use of a main sample acquisition pump which takes the water from an analysis water inlet, passes through a double "T" fitting to which two valves are also connected (in connection with cleaning water inlets, after the outlet of the double "T" fitting there is a spectrophotometry sensor connected to the analysis syringe and continuing through the analysis water outlet pipe to an analysis water outlet.

The analysis syringe has a number of structural features such that it can be cleaned internally by means of a plunger adjustable to the inner walls of the analysis syringe by means of a plunger tensioning mechanism and an internal disinfection ring based on the use of UV LED lights.

The plunger comprises two flexible rubber bands placed at the ends and a rigid rubber band in the middle, adjustable by means of a tensioning mechanism based on the use of two motors driving pinions which mesh on an adjustment gear.

Fluorimetry, broadly speaking, consists in that when the sample is irradiated at a certain wavelength, due to its physicochemical properties, this sample emits light at different wavelengths. This light emitted by the sample is measured at 90° to the main beam, in order to avoid detecting the wavelengths irradiated on the sample, i.e. those coming from the wavelength generator block. The present invention extends the range of the measurement of the fluorimetric response of scattered light by adding the 270° measurement in addition to the 90° measurement. This increase in the field of view of the measurement allows the measurement to be more robust. Therefore, this measure improves the reflected light collection capacity and the capacity of the fluorophores of the samples in two different positions and not only at 90° as is usual in other existing pieces of equipment.

To carry out the analysis, a different process is used depending on whether the wavelengths to be irradiated belong to the ultraviolet region of the spectrum or, on the contrary, to the visible-infrared region.

The fluorimetric analysis subsystem comprises a disc with concentric LED lights, each emitting at different wavelengths, which are aligned with the optical fibre until they impact on the sample. The block has two movements, one of rotation and the other of vertical displacement to align the concentrical LEDs, the subsystem comprises a toothed disc with a multitude of optical filters on its periphery which are aligned with the sample and the fluorimetry sensor, so that the wavelengths that will pass through the filters are restricted, which makes it possible to know what wavelengths the sample is emitting at any given moment.

Due to the features described above, the following is achieved:
- Prediction of emerging organic pollutants and pathogenic micro-organisms by combining spectrophotometry transmittance and absorbance and fluorimetry transmittance measurements and using them in combination to perform statistical regressions with a statistically significant p-value < 0.001, wherein said p-value is defined as the probability that a calculated statistical value is possible given a certain null hypothesis.
- It allows a volume of water to be stored and isolated for multiple spectrofluorescence analyses to establish the indirect correlation with the concentration of emerging organic pollutants, organic matter, pathogenic micro-organisms, as well as particle size present in the water.
- Continuous and real-time analysis of the pollutant load of the water, both in dissolved and particulate compounds that have some kind of influence on the spectral response. In other words, wherein the spectral response emitted by the sample against the excitation received by the equipment, in terms of transmittance recorded at 180^{or} and at 90^{or} and 270°, yields values that, when statistically analysed, allow a significant relationship to be established with the analytes or compounds existing in the water matrix.
- Simultaneous spectrophotometric and molecular fluorescence analysis using LED technology
- Analysis unit with the ability to self-clean and prevent the accumulation of elements on the interior walls.
- The invention can disinfect the inside of the analysis block by means of a quartz-coated inner ring with UV LEDs arranged radially therein, so that the flow is not affected.
- The water passes through the interior of the analysis block, eliminating the need to move the plunger for acquisition and increasing its service life.
- Automatic system for the clamping of the plunger of the analysis block.
- Modular design
- The analysis block can control the sample temperature through the use of Peltier cells.

Except when indicated otherwise, all of the technical and scientific elements used in this specification have the meaning commonly understood by a person with average skill in the art to which this invention belongs. When this invention is put into practice, methods and materials may be used that are similar or equivalent to the ones described in this specification.

Throughout the description and in the claims, the word "comprises" and its variants are not intended to exclude other technical characteristics, additives, components or steps. For those skilled in the art, other objects, advantages and features of the invention may be deduced from both the description and the practical use of the invention.

### EXPLANATION OF THE FIGURES

As a complement to the present description, and for the purpose of helping to make the features of the invention more readily understandable, in accordance with a preferred practical exemplary embodiment thereof, said description is accompanied by a set of drawings constituting an integral part of the same, which by way of illustration and not limitation represent the following.

In figures 1A and 1B, we can observe a representation of the front view and the rear view of the equipment object of the invention.
Figure 2 shows a representation of the modular structure of the equipment.
Figure 3 shows a detail of how the modules of the modular structure are closed by means of rectangular closing platforms.
Figures 4A and 4B show the front and rear views of the equipment.
Figures 5A and 5B show two different positions of the plunger of the analysis syringe when the lifting platform is moved.
Figure 6A shows a representation of the lifting platform.
Figure 6B shows the support block for the upper and lower Hall effect sensors.
Figure 7 shows structural details of the analysis syringe.
Figure 8 shows the sleeve of the analysis syringe.
Figure 9 shows an exploded view of the elements of the threaded inner rod of the analysis syringe.
Figure 10 shows the analysis blocks arranged around the analysis syringe.
Figure 11 shows an exploded view of the elements associated with the analysis blocks, showing the input of the excitation wavelengths, as well as the main spectrophotometric analysis sensor and the fibre socket for capturing the scattered light at 270°.
Figures 12A and 12B show an unexploded and exploded view of the plunger tensioning block.
Figure 13 shows a detail of the location of the fluorimetric analysis subsystem, showing the emission spectrum selection block at 90 and 270°.
Figure 14 shows an exploded view of the fluorimetric analysis subsystem.
Figures 15A and 15B show details of the modular control electronics subsystem.
Figure 16 shows how the printed circuit boards are removed.
Figure 17 shows the power supply subsystem.
Figure 18 shows the location of a secondary pump for distilled water propulsion.
Figure 19 shows the mechanism of the scattered light selection block for fluorimetric analysis.

### PREFERRED EMBODIMENT OF THE INVENTION

With reference to the figures, a preferred embodiment of the invention proposed is described below.

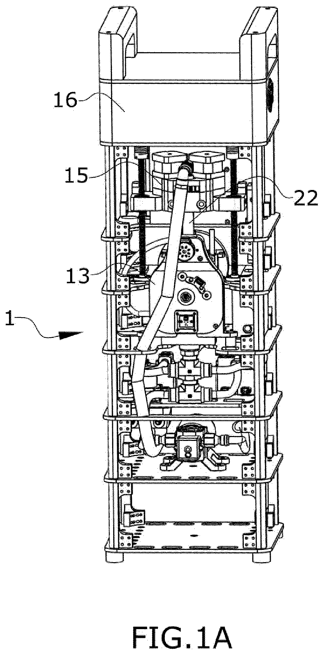

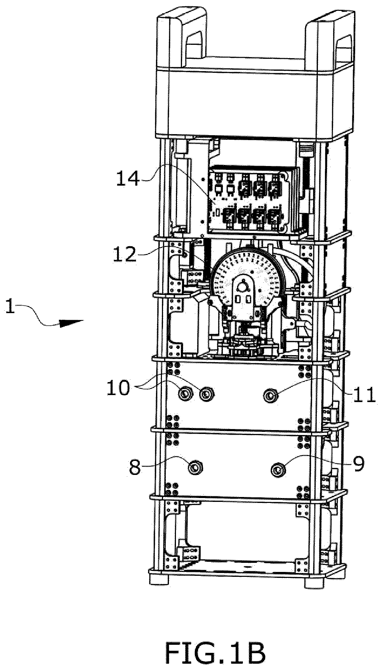

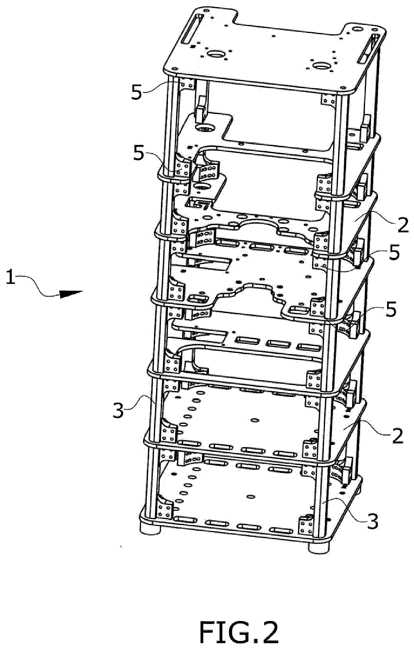

Figure 1 shows that the equipment comprises a modular structure (1), while figure 2 shows how the modular structure (1) comprises a series of interconnected platforms (2) by means of threaded rods, not shown, vertical spacers (3) being arranged between the interconnected platforms (2) that allow the interior space to be divided into different modules.

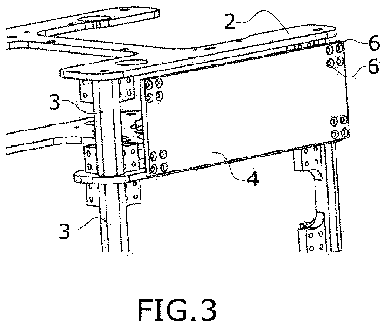

Access to each of the modules defined by the modular structure is covered by rectangular closing platforms (4), as shown in Figure 3, which are attached to the modular structure (1) by means of brackets (5) provided with holes corresponding to the holes (6) arranged on the rectangular closing platforms (4).

Figure 1B shows the water inlet (10) for cleaning as well as the water outlet (11) arranged on one module, while on another module there is a water inlet for analysis (8) and a water outlet for analysis (9).

It should be noted that the modular structure (1) comprises:
- a wavelength generation subsystem (12),
- a fluorimetric analysis subsystem (13),
- a scattered light selection sub-system between 90° and 270° or scattered light selector block (90),
- a control electronics subsystem (14),
- a power supply subsystem and plunger lift motors (16); and
- an analysis syringe (22), comprising:
   ∘ means of mechanical cleaning consisting of vertical displacement of the analysis syringe (22) in association with vertical positioning means of the mechanical cleaning mechanism of the syringe.
   ∘ cleaning means consisting of passing clean water through the interior of the analysis syringe (22).
   ∘ cleaning means for cleaning the inner walls of the analysis walls by means of UV LED lights in combination with plunger tensioning means (15).

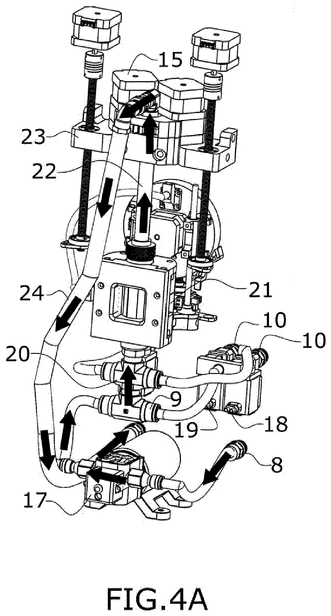

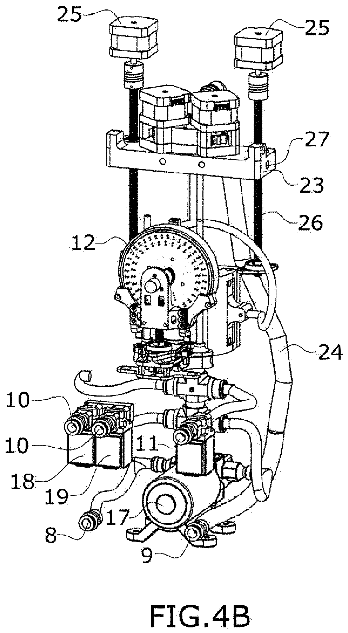

Figures 4A and 4B show the following additional elements: a main sample acquisition pump (17) which takes water from the analysis water inlet (8), passes through a double "T" fitting (20) to which two valves (18) and (19) are also connected in connection with the water inlets (10) for cleaning, after the outlet of the double "T" fitting (20) there is a spectrophotometric sensor (21) connected to the analysis syringe (22), the water flowing in the direction of the arrows and continuing through the outlet pipe (24) of the analysed water to the analysis water outlet (9).

Above the analysis syringe (22) is a lifting and lowering platform (23) as well as the syringe plunger tensioning block.

As indicated above, the analysis syringe (22) has three means or ways of being cleaned:
- Mechanical cleaning means consisting of the vertical displacement of the analysis syringe rod associated with means of vertical positioning of the mechanical cleaning mechanism of the syringe.
- Means through the circulation of clean water through the interior of the tank.
- Cleaning means based on the use of a disc of UV LED lights arranged radially on the end of the plunger rod, in combination with plunger tensioning means.

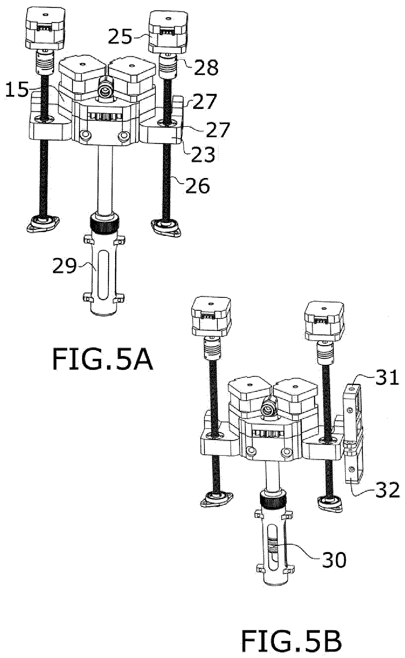

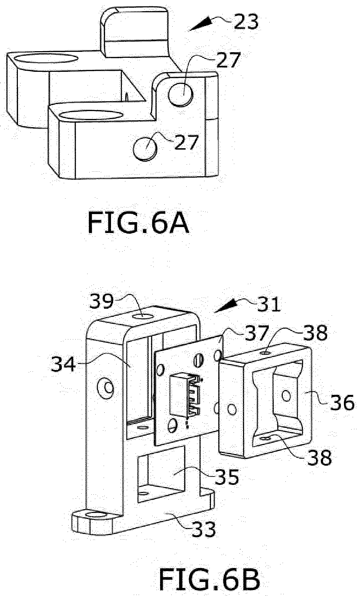

Figures 5A, 5B, 6A and 6B show the mechanical means used for cleaning the analysis syringe (22) comprising:
- A lifting platform (23) running vertically along threaded spindles (26) driven by lifting motors (25) mounted at the upper end of the threaded spindles (26) by means of couplings (28).
- Neodymium magnets (27) arranged on the lifting platform (23) for correct vertical positioning.
- Two limit switch blocks based on Hall effect. There are two on each side of the platform, but for the sake of simplicity in Figure 5, only those on the right-hand side are shown, thus having an upper limit switch (31) and a lower limit switch (32).

The upper limit switch (31) is arranged on a support structure (33) with an upper window (34) and a lower window (35), so that a junction block (36) of a PCB integrated circuit board (37) of the limit switch is arranged on the upper window (34), said junction block having a threaded hole (38) through which it is fixed to the support structure (33) by means of a screw that goes through the hole (39) arranged in the support structure (33), as shown in figure 6B.

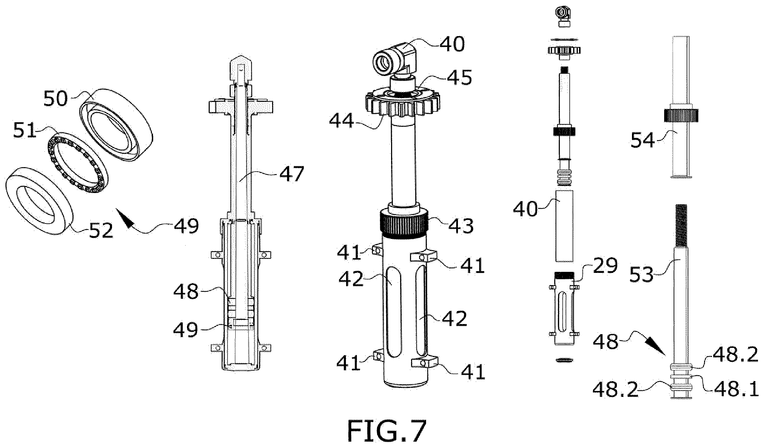

Figure 7 shows the structural features of the analysis syringe (22) which, as can be seen, comprises a sleeve (29) provided with a series of windows (42), inside of which a glass (40) is housed, said sleeve (29) having two pairs of fastening tabs (41), an upper connecting thread (43) is arranged above the sleeve (29) and at the upper end of the rod there is a gear (44) for adjusting the plunger rubbers (48), wherein said gear (44) has an axial bearing (45) so that it can be passed through by an elbow (46).

Below the plunger rubbers (48) there is an inner disinfection ring (49), comprising:
- an outer ring casing (50)
- a disc of UV LED lights (51) housed in said outer ring casing (50)
- a transparent quartz shield (52) which is arranged over the UV LED light disc (52).

The plunger rubbers (48) in turn comprise two flexible rubbers (48.1) positioned at the ends and an intermediate stiff rubber (48.2).

The analysis syringe rod is a rod with an inner channel (47) for the passage of waste water inside the syringe and comprises a threaded inner rod (53) and an outer rod (54).

Both rods have a flat circular surface at the end with a diameter somewhat smaller than that of the plunger, which is what produces the buckle thereof.

To control the tightness of the assembly, and therefore the degree of adjustment of the syringe, this is done by screwing a gear located in the upper portion of the assembly onto the thread of the inner rod, which causes the inner and outer rods to come closer together, causing the deformation of the plunger rubbers (48) and therefore the tightness of the syringe.

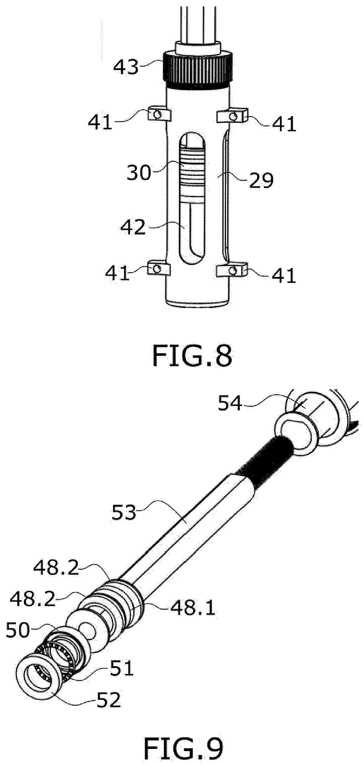

Figure 9 shows an exploded view of the elements described above, showing the plunger rubbers (48) as well as the inner disinfection ring (49).

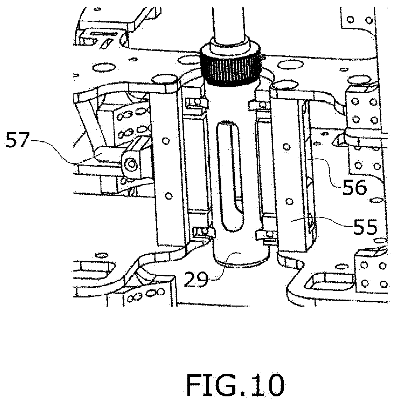

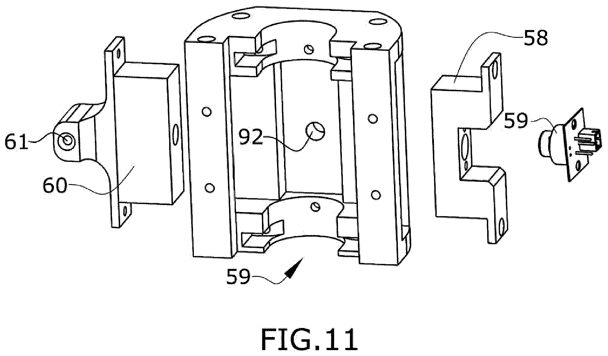

Figures 10 and 11 show the means used for connecting the fibre optic sensor and the spectrophotometry sensor to an analysis block (55) which is provided on one of its sides with a housing (56) to arrange a spectrophotometry sensor holder (58) to which the spectrophotometry sensor (59) is attached, while on the opposite side to that of the analysis block (55) described above there is an input holder (60) for an optical fibre, said holder (60) having a housing for a grub screw (61) and an input (57) for an optical fibre of the excitation wavelengths. Furthermore, the analysis block (55) has an opening (92) located at 270° with respect to the excitation wavelengths optical fibre input (57), intended to house an additional optical fibre (91) responsible for capturing the emission wavelengths coming from one side of the analysis syringe (22), and redirecting it towards the scattered light selector block (90).

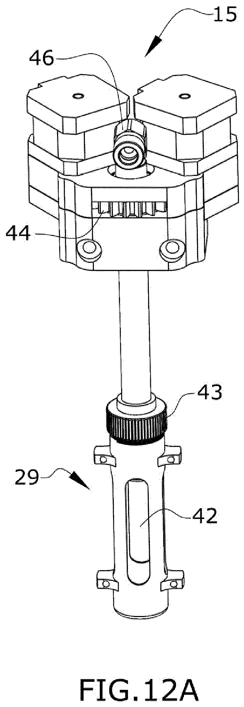

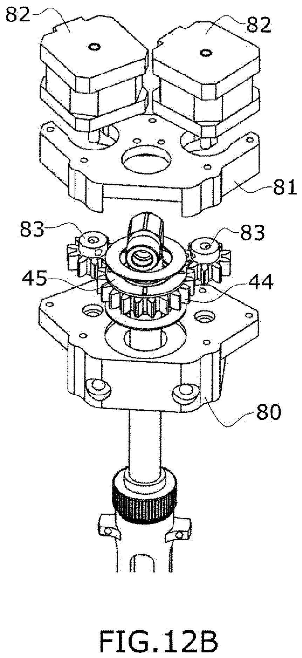

Figures 12A and 12B show the plunger tensioning block (15) which, as can be seen, comprises a casing formed by a lower tensioner cover (80), on which an upper cover (81) is arranged and in the space defined between both covers there is the adjustment gear (44) of the plunger (33), wherein said adjustment gear (44) is driven by two pinions (83) driven by plunger tensioning motors (82) placed on the upper cover (81).

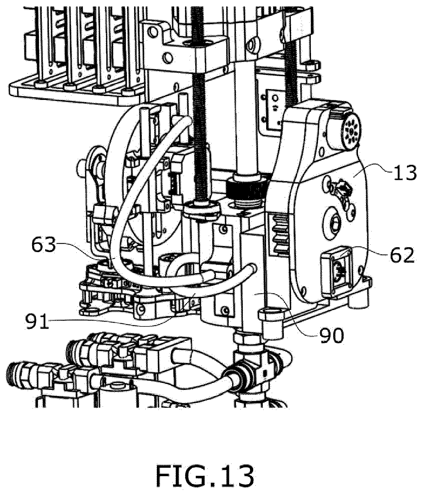

Figure 13 shows the fluorimetric analysis subsystem (13) which, as can be seen, has a fluorimetric sensor (62) and an optical fibre (63) connected thereto.

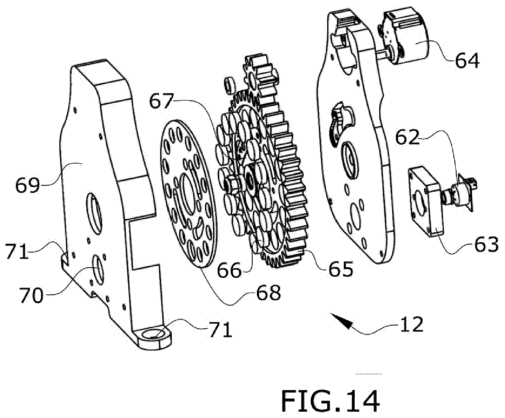

Figure 14 shows the wavelength generation subsystem (12) which, as can be seen, comprises a toothed wheel (65) on which a series of optical filters (66) and alignment Hall sensors (67) are arranged, an optical filter cover (68) being arranged on top of the previous assembly (68), all of the above elements being housed in a casing formed by a cover (69) provided with a light inlet (70) coming from the syringe, and said cover (69) having fastening screws (71), while the previous facing cover has the connection of a drive motor (64), a support block (63) and a fluorimetric sensor (62).

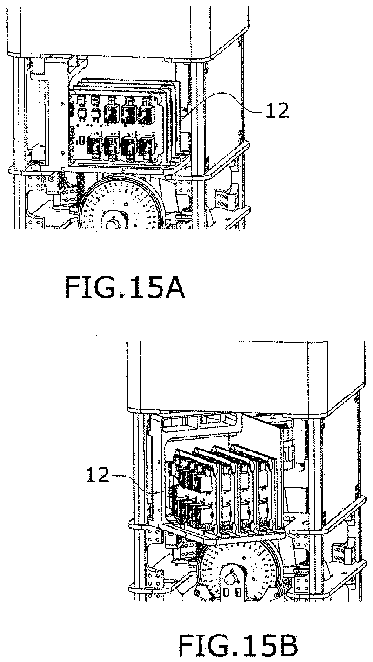

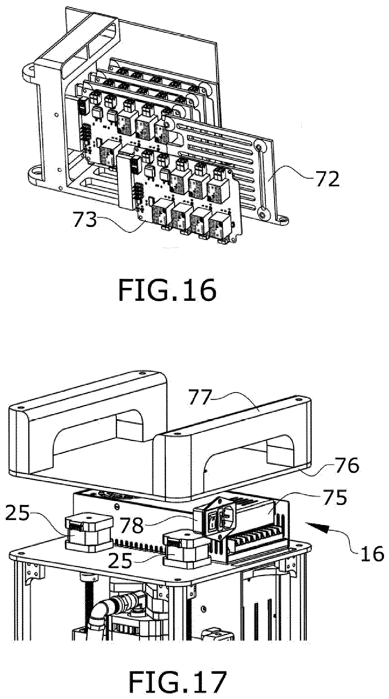

Figures 15A and 15B show a detail view of the modular control electronics subsystem (14), which as can be seen in Figure 16 has a series of removable bays (72) to which the PCB boards (73) are attached.

Figure 17 shows a detail view of the power supply subsystem (16), which houses therein the power supply itself (75), an electrical socket (74) all of this being covered by a cover (76) provided with handles (77).

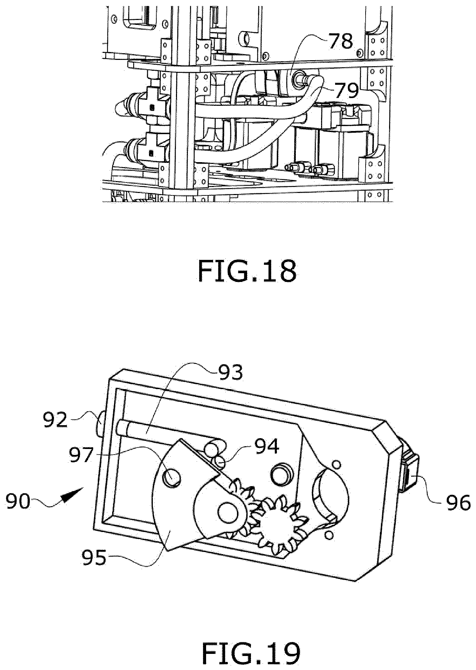

Figure 18 shows a detail view of a secondary pump (78) for distilled water, which is required to calibrate the equipment. Said secondary pump (78) takes the water from a tank located at the lower portion of the equipment and introduces it through the double "T" fitting (20) (Figures 4A).

Figure 19 shows the exploded view of the scattered light selector block (90), consisting of a receptacle into which the fluorimetric analysis optical fibre (91) from the analysis block (55) is inserted via a 270° scattered light inlet (92) to capture the emission wavelengths produced at 270° with respect to the excitation wavelengths optical fibre inlet (57). This optical fibre is continued inside the scattered light selection block (90) by a straight section of inner 270° scattered light optical fibre (93) until it is positioned next to the 90° scattered light input (94) which consists of a straight optical fibre for better light collection and redirection. The scattered light selector block (90) comprises a motorised system driven by a motor (96) to move a tilting scattered light selector (95) which has an orifice (97) that aligns with only one of the fibre inputs, either the 270° scattered light optical fibre input (92) and the inner optical fibre channel (93) or the 90° scattered light inner optical fibre input (94) to allow only one of them to pass into the fluorimetric analysis block (13).

Having thus adequately described the nature of the present invention, as well as how to put it into practice, it must be noted that, within its essential nature, the invention may be carried out according to other embodiments differing in detail from that set out by way of example, which the protection sought would equally cover, provided that the fundamental principle thereof is not altered, changed or modified.

## Claims

1. An improved equipment for performing spectrophotometry and fluorimetry, **characterised in that** it comprises a modular structure (1) on which the following are arranged:
- a wavelength generation subsystem (12),
- a fluorimetric analysis subsystem with an extended range of 90° and 270° (13),
- a scattered light selection subsystem between 90° and 270° (90),
- a control electronics subsystem (14),
- a power supply subsystem and plunger lift motors (16),
- an analysis syringe (22) provided with three cleaning means, namely:
∘ means of mechanical cleaning consisting of vertical displacement of the analysis syringe (22) in association with vertical positioning means of the mechanical cleaning mechanism of the syringe,
∘ cleaning means consisting of passing clean water through the interior of the analysis syringe (22),
∘ cleaning means for cleaning the inner walls of the analysis walls by means of UV LED lights in combination with plunger tensioning means (15),
wherein the mechanical cleaning means for cleaning the analysis syringe (22) comprise:
- a lifting platform (23) running vertically along threaded spindles (26) driven by lifting motors (25) mounted at the upper end of the threaded spindles (26) by means of couplings (28),
- neodymium magnets (27) arranged on the lifting platform (23) for correct vertical positioning,
- two Hall-effect based limit switch blocks with an upper limit switch (31) and a lower limit switch (32).

2. The improved equipment for performing spectrophotometry and fluorimetry according to claim 1, **characterised in that** the modular structure (1) comprises a series of platforms interconnected (2) by means of threaded rods, vertical spacers (3) being arranged between the interconnected platforms (2), wherein each of the modules defined by the modular structure is covered by rectangular closing platforms (4).

3. The improved equipment for performing spectrophotometry and fluorimetry according to claim 1 or 2, **characterised in that** it comprises a scattered light selector block (90), which allows the emission wavelengths to pass towards the fluorimetric analysis block (13), which allows the light taken at 270° to pass through and conducted by means of optical fibre to the scattered light selector block (90).

4. The improved equipment for performing spectrophotometry and fluorimetry according to claim 3, **characterised in that** the scattered light selector block (90) consists of a receptacle having a scattered light inlet measured at 270° (92) from the analysis syringe (22) by means of a socket present in the analysis block (55), as well as an inlet for scattered light at 90° (94) from the analysis syringe (22), and a scattered light selector (95) driven by a motor (96) fitted with an orifice (97), so that when rotated, it covers one of the scattered light collection sockets, causing only one of them to reach the fluorimetric analysis block (13).

5. The improved equipment for performing spectrophotometry and fluorimetry according to any of the preceding claims, **characterised in that** the upper limit switch (31) is arranged on a support structure (33) with an upper window (34) and a lower window (35), so that a junction block (36) of a PCB integrated circuit board (37) of the limit switch is arranged on the upper window (34), said junction block having a threaded hole (38) through which it is attached to the support structure (33) by means of a screw that goes through the hole (39) arranged in the support structure (39).

6. The improved equipment for performing spectrophotometry and fluorimetry according to any of the preceding claims, **characterised in that** the cleaning means consisting of passing clean water through the inside of the analysis syringe (22) comprise: a main sample acquisition pump (17) which takes water from an analysis water inlet (8), passes through a double "T" fitting (20) to which two valves (18) and (19) are also connected in connection with water inlets (10) for cleaning, after the outlet of the double "T" fitting (20) there is a spectrophotometric sensor (21) connected to the analysis syringe (22), the water flowing in the direction of the arrows and continuing through the outlet pipe (24) of the analysed water to an analysis water outlet (9).

7. The improved equipment for performing spectrophotometry and fluorimetry according to any of the preceding claims, **characterised in that** the analysis syringe (22) comprises a sleeve (29) provided with a series of windows (42), inside of which a glass (40) is housed, said sleeve (29) having two pairs of fastening tabs (41), an upper connecting thread (43) is arranged above the sleeve (29) and at the upper end of the plunger rod there is a plunger tensioning block (15) for adjusting the plunger rubbers (48), wherein said gear (44) has an axial bearing (45) so that it can be crossed by an elbow (46), while below the plunger rubbers (48) there is an inner disinfection ring (49).

8. The improved equipment for performing spectrophotometry and fluorimetry according to claim 7, **characterised in that** the inner disinfection ring (49) comprises:
- an outer ring casing (50)
- a disc of UV LED lights (51) housed in said outer ring casing (50)
- a transparent quartz shield (52) which is arranged over the UV LED light disc (52).

9. The improved equipment for performing spectrophotometry and fluorimetry according to claim 7, **characterised in that** the plunger rubbers (48) in turn comprise two flexible rubbers (48.1) placed at the ends and an intermediate rigid rubber (48.2).

10. The improved equipment for performing spectrophotometry and fluorimetry according to claim 7 or 9, **characterised in that** the plunger tensioning block (15) comprises a casing formed by a lower tensioner cover (80), on which an upper cover (81) is arranged and in the space defined between both covers there is the adjustment gear (44) of the plunger (33), wherein said adjustment gear (44) is driven by two pinions (83) driven by plunger tensioning motors (82) placed on the upper cover (81).
